# EUROPEAN PATENT APPLICATION

(11) **EP 4 803 197 A2**
(43) Date of publication of application: **09.09.2026**
(21) Application number: 26162444.9
(22) Date of filing: 04.03.2026
(51) Int. Cl.: B01L 3/00, G01N 21/03, G01N 33/49, G01N 21/05

(54) **MEDICAL FLOW-THROUGH CUVETTE AND LIQUID DETECTION DEVICE**

(30) Priority: 05.03.2025 CN 202510256638
(71) Applicant: Lifemotion Medical Technology Co., Ltd., Shenzhen, Guangdong 518126 (CN)
(72) Inventor: LIU, Gaojian, Shenzhen Guangdong, 518126 (CN)
(74) Representative: Pfenning, Meinig & Partner mbB

(57) **Abstract**

The present application provides a medical flow-through cuvette, one side wall of a flow-through cuvette body is a planar structure, and a cross-section of the flow-through cuvette body corresponding to the planar structure is different from cross-sections of the flow-through cuvette body; the planar structure part can be used for detector detection. The present application further provides a blood liquid detection device, which covers the flow-through cuvette with a shell in the device to avoid interference from external light on internal light reception, and realizes blood liquid detection by arranging a photoelectric emitting device and a photoelectric receiving device on the same side

## Description

### Technical Field

The embodiments of the present application relate to the technical field of medical equipment, and more particularly to a medical flow-through cuvette and a liquid detection device.

### Background Art

During the emergency treatment of critically ill patients with severe cardiopulmonary failure, extracorporeal membrane oxygenation (ECMO) is used to provide continuous extracorporeal respiratory and circulatory support to buy more valuable time for treatment. In the implementation of existing ECMO systems, the detection of blood oxygen saturation and hematocrit requires manual blood sampling and analysis. The medical flow-through cuvette installed on existing detection devices lacks an anti-reverse installation design, so medical personnel may install the medical flow-through cuvette in the wrong direction when attaching it to the detection device, leading to errors in the detection device's analysis of the liquid in the medical flow-through cuvette.

### Summary of the Invention

To address or mitigate the technical problems in the existing technology, in a first aspect, the embodiments of the present application provide a medical flow-through cuvette, comprising:
a flow-through cuvette body, wherein two fixed stop members are arranged at intervals at an end of the flow-through cuvette body;
one side wall of the flow-through cuvette body between the two fixed stop members is a planar structure, and a cross-section of the flow-through cuvette body corresponding to the planar structure is different from other cross-sections of the flow-through cuvette body, wherein a detector is attached to a side of the planar structure away from the flow-through cuvette body, and the detector is used to detect a liquid in the flow-through cuvette body.

As a preferred embodiment of the present application, a cross-section of the fixed stop member is larger than a cross-section of an end of the flow-through cuvette body connected to the fixed stop member.

As a preferred embodiment of the present application, the flow-through cuvette body is provided with at least one first groove on a side close to the detector, the first groove is arranged along an extension direction of the flow-through cuvette, and the first groove is arranged on a periphery of the detector.

Compared with the existing technology, the medical flow-through cuvette provided by the embodiments of the present application comprises a flow-through cuvette body, with two fixed stop members arranged at intervals at the end of the flow-through cuvette body; one side wall of the flow-through cuvette body between the two fixed stop members is a planar structure, and the cross-section of the flow-through cuvette body corresponding to the planar structure is different from other cross-sections of the flow-through cuvette body, a detector is attached to the side of the planar structure away from the flow-through cuvette body, and the detector is used to detect the liquid in the flow-through cuvette body. Through the design of this flow-through cuvette, the technical problems in the existing technology can be addressed, ensuring that medical personnel do not install the medical flow-through cuvette incorrectly when attaching it to the detection device, thereby improving the detection accuracy of the detection device.

In a second aspect, the embodiments of the present application further provide a medical blood liquid detection device, characterized in that the device comprises a detector and the medical flow-through cuvette according to any items of the first aspect;
the detector comprises a shell and a detection unit arranged in the shell, wherein the detection unit detects parameters of the liquid in the flow-through cuvette;
an upper surface of the shell is provided with a second groove and a fixing mechanism, the second groove is configured to install the flow-through cuvette; one end of the fixing mechanism is movably connected to one side of the second groove, another end of the fixing mechanism is rotatable around the one end of the fixing mechanism, and the fixing mechanism covers an upper surface of the flow-through cuvette;
when the cross-section of the flow-through cuvette body corresponding to the planar structure is larger than the other cross-sections of the flow-through cuvette body, a third groove is provided in the second groove, and the planar structure on one side of the flow-through cuvette body cooperates with the third groove to fix the flow-through cuvette;
when the cross-section of the flow-through cuvette body corresponding to the planar structure is smaller than the other cross-sections of the flow-through cuvette body, a boss is provided in the second groove, and the planar structure on one side of the flow-through cuvette body cooperates with the boss to fix the flow-through cuvette.

As a preferred embodiment of the present application, the detection unit comprises a photoelectric transmitter and a photoelectric receiver;
a first chamber and a second chamber are sequentially arranged at intervals on the same side within a lower surface shell of the flow-through cuvette;
the first chamber is provided with the photoelectric transmitter, and the second chamber is provided with the photoelectric receiver.

As a preferred embodiment of the present application, the detection unit further comprises an infrared detector;
a third chamber is further provided within the shell, the third chamber is arranged adjacent to and spaced apart from the second chamber, and the infrared detector is provided in the third chamber;
a distance between the third chamber and the second chamber is different from a distance between the second chamber and the first chamber.

As a preferred embodiment of the present application, an inner top of the first chamber, an inner top of the second chamber and an inner top of the third chamber are respectively correspondingly provided with a first transparent protective member, a second transparent protective member and a third transparent protective member, and the first transparent protective member, the second transparent protective member and the third transparent protective member are configured to prevent foreign objects from falling into the first chamber, the second chamber and the third chamber, respectively.

As a preferred embodiment of the present application, a length of the photoelectric transmitter and a length of the photoelectric receiver along a blood flow direction of the flow-through cuvette are the same, and a ratio of a center distance between the photoelectric transmitter and the photoelectric receiver to the length is between 1.1 and 1.5.

As a preferred embodiment of the present application, an elastic member is provided on a side of the fixing mechanism opposing the flow-through cuvette; when the flow-through cuvette is fixed by the fixing mechanism, the elastic member contacts the flow-through cuvette and undergoes elastic deformation to secure the flow-through cuvette.

As a preferred embodiment of the present application, fourth grooves are provided at ends of the shell at positions corresponding to the stop members, with a portion of the stop members being disposed in the corresponding fourth grooves to secure the flow-through cuvette, and cross-sections of the two stop members gradually decrease in a downward direction along the fourth grooves.

Compared with the existing technology, the present application provides a blood liquid detection device, which comprises a first fixing structure that can rotate around one end of a first fixing mechanism on the upper surface of a flow-through cuvette, covering the flow-through cuvette to prevent interference from external light on internal light reception, and further securing the flow-through cuvette through the first fixing structure to avoid measurement errors caused by movement during blood measurement.

### Brief Description of the Drawings

The drawings described herein are provided to further aid the understanding of the present application and constitute a part of the present application. The illustrative embodiments and their descriptions in the present application are used to explain the present application and do not constitute undue limitations on the present application. Some specific embodiments of the present application will be described in detail below by way of example rather than limitation with reference to the drawings. In the drawings, the same reference numerals indicate the same or similar components or parts. It should be understood by a person skilled in the art that these drawings are not necessarily drawn to scale. In the drawings:
FIG. 1 shows a structural diagram of a flow-through cuvette (e.g., a medical pipeline or tubing).
FIG. 2 shows a perspective structural diagram of a blood detection device.
FIG. 3 shows a top view structural diagram of the blood detection device (i.e., of FIG. 2).
FIG. 4 shows an A-A cross-sectional view of FIG. 3.
FIG. 5 shows a partial enlarged view of FIG. 4.
FIG. 6 shows a B-B cross-sectional view of FIG. 3.
FIG. 7 shows a partial enlarged view of FIG. 6.

The following are the reference numerals in the drawings:
1: cuvette body; 1-1: fixed stop member; 1-2: first grooves; 1-3: planar structure; 2: shell; 2-1: boss; 2-2: fixing mechanism; 2-2-1: observation window; 2-2-2: elastic member; 2-3: photoelectric transmitter; 2-4: photoelectric receiver; 2-5: infrared detector; 2-6: first transparent protective member; 2-7: second transparent protective member; 2-8: third transparent protective member; 2-9: second groove; 2-10: detection unit; 2-11: first chamber; 2-12: second chamber; 2-13: third chamber; 2-14: third groove.

### Description of the Embodiments

In order to enable a person skilled in the art to better understand the solutions of the present application, the technical solutions in the embodiments of the present application will be clearly and fully described below in conjunction with the drawings in the embodiments of the present application. It is obvious that the described embodiments are only some embodiments of the present application, and not all embodiments. Based on the embodiments in the present application, all other embodiments obtained by a person skilled in the art without inventive effort should fall within the scope of protection of the present application.

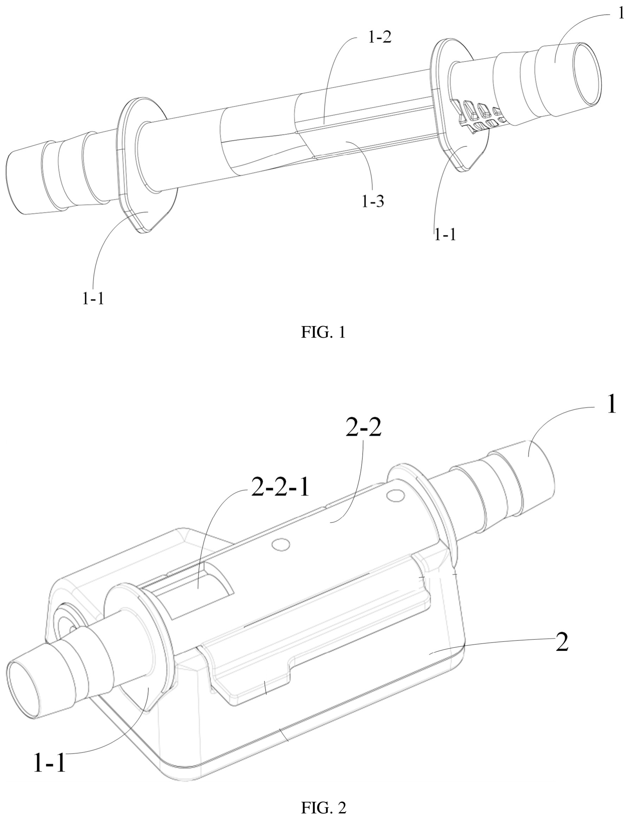

As shown in FIG. 1, the embodiments of the present application provide a medical flow-through cuvette, which comprises:
a cuvette body 1, where two fixed stop members 1-1 are arranged at intervals at an end of the pipeline body;
a side wall of the cuvette body 1 between the two fixed stop members 1-1 is a planar structure 1-3, and the cross-section of the cuvette body 1 corresponding to the planar structure is different from other cross-sections of the cuvette body 1, a detector is attached to a side of the planar structure 1-3 away from the cuvette body 1, and the detector is used to detect a blood liquid in the cuvette body 1.

In some embodiments of the present application, the cross-section of the fixed stop member 1-1 is larger than the cross-section of the end of the cuvette body 1 connected to the fixed stop member 1-1; this allows for better fixation of the cuvette body 1.

In some embodiments of the present application, at least one first groove 1-2 is provided on the side of the cuvette body 1 close to the detector, the first groove 1-2 is arranged along the extension direction of the pipeline, and the first groove 1-2 is arranged on the periphery of the detector. By providing the first groove 1-2, the influence of external light on the detection structure of the detection device can be reduced. The height of the first groove 1-2 accounts for approximately 20% to 80% of the wall thickness of the pipeline in the contact area, and preferably 40% to 60%.

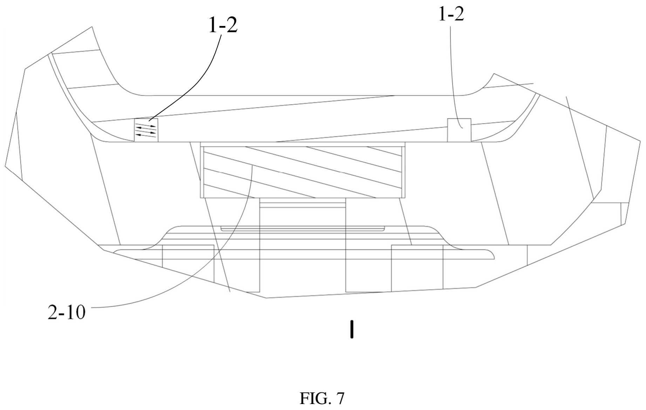

In the embodiments of the present application, the first groove 1-2 is arranged on the periphery of the detection unit. When external light enters the cuvette body 1 through a gap of the shell 2, the multiple first grooves 1-2 at the bottom of the cuvette body 1 can reduce the impact of interfering light on the detection unit's reception of light by reflecting and refracting it. The multiple reflections of light within the first groove 1-2 can be referred to the arrows shown in FIG. 7.

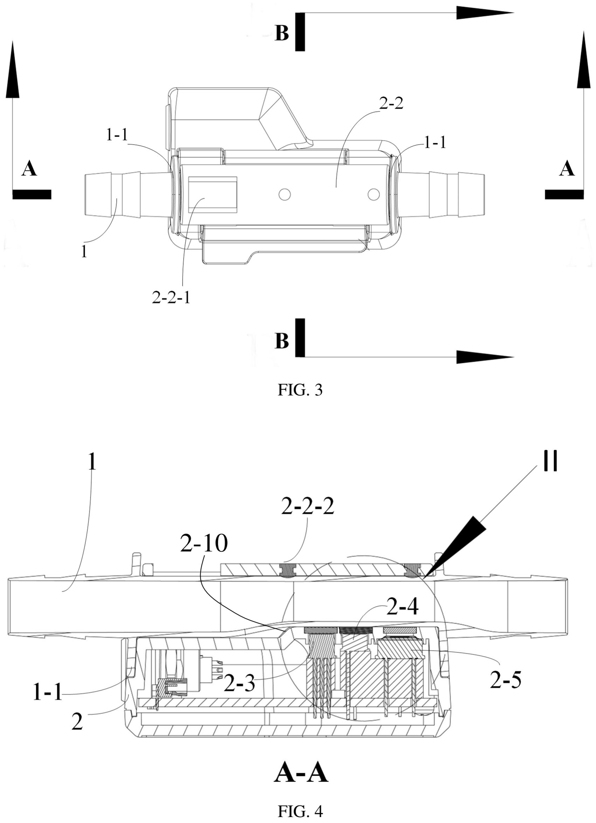

As shown in FIG. 2 and 3, the embodiments of the present application also provides a medical blood liquid detection device, which comprises a detector and the medical flow-through cuvette described in any item of the first aspects.

The detector comprises a shell 2 and a detection unit 2-10 disposed in the shell, the detection unit 2-10 is used to detect parameters of a blood liquid in the flow-through cuvette.

The upper portion of the shell 2 includes a third groove 2-14 and a fixing mechanism 2-2. The third groove 2-14 is used to install the flow-through cuvette. One end of the fixing mechanism 2-2 can movably connect to one side of the third groove 2-14. The other end of the fixing mechanism 2-2 can rotate around the one end of the fixing mechanism 2-2, and the fixing mechanism 2-2 covers the upper surface of the flow-through cuvette.

In the embodiments of the present application, when the cross-section of the cuvette body 1 corresponding to the planar structure 1-3 is larger than the other cross-sections of the cuvette body 1, a third groove (not shown) is provided in the second groove 2-9, and the planar structure on one side of the cuvette body 1 cooperates with the third groove to fix the cuvette.

As shown in FIG. 2, when the cross-section of the cuvette body 1 corresponding to the planar structure 1-3 is smaller than the other cross-sections of the cuvette body 1, a boss 2-1 can be provided in the second groove 2-9, and the planar structure 1-3 on one side of the cuvette body 1 cooperates with the boss 2-1 to fix the cuvette.

The detector 2-10 comprises a photoelectric detection unit and an infrared detection unit, the photoelectric detection unit is used to detect blood oxygen saturation, hematocrit, and hemoglobin, etc., and the infrared detection unit is used to detect the temperature of the blood.

In the embodiments of the present application, the structure of the fixing mechanism 2-2 is an arc-shaped structure, the cuvette body 1 is made of a transparent material, and the fixing mechanism 2-2 covers the upper surface of the cuvette body 1. The fixing mechanism 2-2 can rotate around its other end. When it is necessary to remove the cuvette body 1, the other end of the fixing mechanism 2-2 is rotated around one end of the fixing mechanism 2-2 away from the detection unit 2-10 to remove the cuvette body 1. More specifically, one end of the fixing mechanism 2-2 is connected to the shell 2 via a shaft (not shown), and the other end of the fixing mechanism 2-2 is fixed to the shell 2 via a buckle or slot (e.g., groove 2-14). After the fixing mechanism 2-2 secures the cuvette body 1, the combination of the fixing mechanism 2-2 and the shell 2 covers the surroundings of the cuvette body 1, preventing external light from entering the shell 2 and avoiding interference with the operation of the photoelectric detection unit. Moreover, the fixing structure 2-2 further secures the cuvette body 1 to avoid measurement errors caused by movement during the blood measurement process.

In addition, in the embodiments of the present application, an observation window 2-2-1 is provided on the fixing mechanism 2-2, through which the blood flow in the cuvette body 1 can be observed to enable more accurate blood detection. Specifically, the observation window 2-2-1 is an open structure, and more specifically, the observation window 2-2-1 is arranged in the area of the fixing mechanism 2-2 away from the detection unit to reduce the interference of light passing through the observation window 2-2-1 during the operation of the detection unit 2-10.

In another embodiment of the present application, as shown in FIG. 4, the photoelectric detection unit comprises a photoelectric transmitter 2-3 and a photoelectric receiver 2-4; a boss 2-1 is provided in the second groove 2-9, and a first chamber 2-11, a second chamber 2-12, and a third chamber 2-13 are arranged at intervals in the shell 2 at positions corresponding to the boss 2-1; the cross-section of the cuvette body 1 disposed on the boss 2-1 is smaller than the cross-section of the cuvette body 1 not disposed on the boss 2-1.

The first chamber 2-11 is provided with a photoelectric transmitter 2-3, the second chamber 2-12 is provided with a photoelectric receiver 2-4, and the third chamber 2-13 is provided with an infrared detector 2-5.

In the embodiments of the present application, the photoelectric transmitter 2-3, the photoelectric receiver 2-4 and the infrared detector 2-5 are arranged in different chambers to avoid interference caused by light refraction within the same chamber, thereby enabling more accurate blood measurements. In addition, the second groove 2-9 is arc-shaped to match the cuvette body 1, and a boss 2-1 is provided in the second groove 2-9, allowing the photoelectric detection unit and the infrared detection unit to be arranged in the boss 2-1. The upper surface of the boss 2-1 is planar, and the cuvette body 1 is placed on the boss 2-1, with the surface of the cuvette body 1 in contact with the boss 2-1 being a plane matching the boss 2-1. Since the emission window of the detection unit is planar, the flush arrangement between the boss 2-1 and the cuvette body 1 can minimize the distance therebetween, thereby reducing light attenuation caused by passing through air and avoiding errors due to insufficient light intensity. Furthermore, the arrangement of the boss 2-1 can prevent incorrect positioning of the cuvette body 1 when placed in the second groove 2-9, thereby avoiding inaccurate detection results due to errors in blood flow direction caused by incorrect positioning.

Moreover, it should be noted that, due to manufacturing reasons, the detection unit cannot be made into an arc shape matching the circular shape of the pipeline 1. Therefore, a parallel fit at the contact point is more conducive to measurement, making the measurement more accurate.

In another embodiment of the present application, the first chamber 2-11 and the second chamber 2-12 are sequentially arranged along the blood flow direction in the pipeline 1, and the photoelectric transmitter 2-3 is at the same horizontal height as the photoelectric receiver 2-4. Since the photoelectric transmitter 2-3 is disposed in the first chamber 2-11 and the photoelectric receiver 2-4 is disposed in the second chamber 2-12, it is necessary to position the photoelectric transmitter 2-3 and the photoelectric receiver 2-4 adjacent to each other and at the same horizontal height to ensure that the photoelectric receiver 2-4 can better receive the signal emitted by the photoelectric transmitter 2-3. This arrangement of the photoelectric transmitter 2-3 and the photoelectric receiver 2-4 enables more accurate measurements, avoiding interference caused by light refraction within the same chamber.

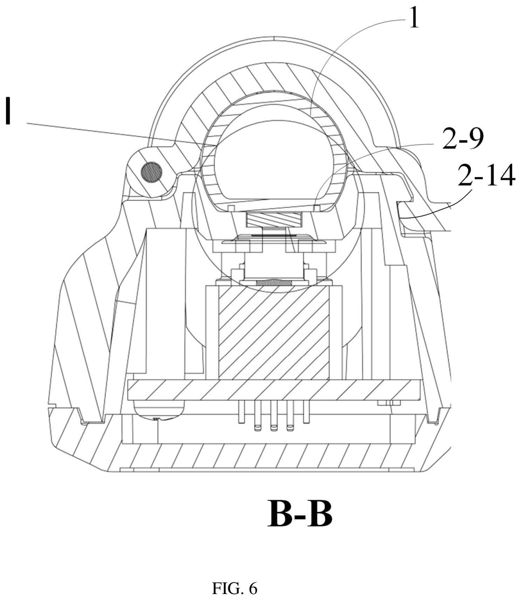

In another embodiment of the present application, as shown in FIG. 4 to 7, the inner top of the first chamber 2-11, the inner top of the second chamber 2-12 and the inner top of the third chamber 2-13 are respectively correspondingly provided with a first transparent protective member 2-6, a second transparent protective member 2-7 and a third transparent protective member 2-8. The first transparent protective member 2-6, the second transparent protective member 2-7 and the third transparent protective member 2-8 are respectively used to prevent foreign objects from falling into the first chamber 2-11, the second chamber 2-12 and the third chamber 2-13. In addition, the infrared detector 2-5 performs temperature detection of the blood in the pipeline 1 by emitting infrared light through the third transparent protective member 2-8 and the pipeline 1. The photoelectric transmitter 2-3 emits a photoelectric signal, which passes through the first transparent protective member 2-6 and the blood in the pipeline 1 and is then received by the photoelectric receiver 2-4 to detect the blood oxygen saturation of the blood in the pipeline 1.

That is to say, a transparent protective member is provided above each chamber. The transparent protective member can be made of transparent materials such as glass (PMMA) or PC board, with glass being preferred. The transparent protective member and the shell 2 can be fixed by ultrasonic welding, integral molding, or bonding. If the first transparent protective member 2-6, the second transparent protective member 2-7 and the third transparent protective member 2-8 are fixed in the shell 2 by bonding, they need to be respectively fixed in the shell through corresponding mounting slots (not shown).

In the present application, the protective member is set to be transparent mainly to avoid the protective member affecting the internal signal transmission between the photoelectric transmitter 2-3 and the photoelectric receiver 2-4.

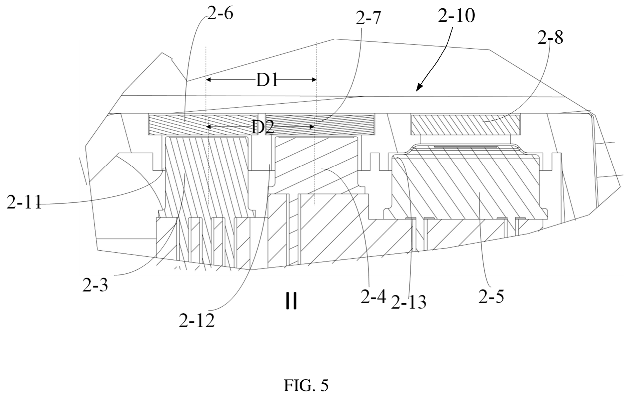

In another embodiment of the present application, as shown in FIG. 5, the center distance D2 between the photoelectric transmitter and the photoelectric receiver 2-4 is smaller than the center distance D1 between the first transparent protective member 2-6 and the second transparent protective member 2-7.

That is to say, the distance between the photoelectric transmitter 2-3 and the photoelectric receiver 2-4 is relatively smaller than the distance between the first transparent protective member 2-6 and the second transparent protective member 2-7. By setting it this way, the light transmission distance is shorter, and the interference is reduced, thereby further improving the accuracy.

In another embodiment of the present application, the photoelectric transmitter 2-3 and the photoelectric receiver 2-4 are arranged along the blood flow direction of the cuvette body 1 and have the same diameter, and the ratio of the center distance between the photoelectric transmitter 2-3 and the photoelectric receiver 2-4 to the diameter is between 1.1 and 1.5. This allows the photoelectric receiver 2-4 to better receive the signals emitted by the photoelectric transmitter 2-3.

In another embodiment of the present application, the fixing mechanism 2-2 is provided with an elastic member 2-2-2 on the side opposing the cuvette body 1; when the cuvette body 1 is fixed by the fixing mechanism 2-2, the elastic member 2-2-2 contacts the cuvette body 1 and undergoes elastic deformation to fix the cuvette body 1.

In the embodiments of the present application, the top of the fixing mechanism 2-2 is provided with an elastic member 2-2-2, and after the first fixing mechanism 2-2 fixes the cuvette body 1, the elastic member 2-2-2 comes into contact with the cuvette body 1. After the cuvette body 1 is fixed in the second groove 2-9 by the first fixing mechanism 2-2, the elastic member 2-2-2 can further secure the cuvette body 1, preventing measurement deviations due to upward movement of the cuvette body 1 caused by vibration or other issues. When specifically detecting blood parameters, during the process of placing the cuvette body 1 into the second groove 2-9, the stop members 1-1 on both sides of the cuvette body 1 slide into the designated positions along the second groove 2-9 to complete the preliminary fixing of the cuvette body 1. At this point, the flat portion of the cuvette body 1 fits against the protrusion. Subsequently, the fixing mechanism 2-2 is flipped and engaged with the shell 2. The elastic member 2-2-2 at the top of the fixing mechanism 2-2 has a certain degree of elastic expansion and contraction capability. After the fixing mechanism 2-2 is engaged, the elastic member 2-2-2 abuts against the cuvette body 1 and undergoes a certain degree of elastic deformation to achieve the fixation of the cuvette body 1.

In some embodiments of the present application, the cuvette body 1 is provided with two fixed stop members 1-1, and an end of the shell 2 is provided with fourth grooves at positions corresponding to the fixed stop members 1-1. Part of the fixed stop members 1-1 is disposed in the corresponding fourth grooves to fix the cuvette body 1. The cross-sectional area of the two fixed stop members 1-1 gradually decreases in the downward direction along the fourth grooves. In addition, the stop members arranged on both sides of the shell 2 provide shielding from both sides, which can also reduce the impact of external light on the detection structure of the detection device.

In the embodiments of the present application, by providing the fixed stop members 1-1 on the cuvette body 1, the cuvette body 1 can be further secured in the second groove 2-9. The distance between the two fixed stop members 1-1 is equal to the length of the shell 2 along the cuvette body 1. By engaging part of the fixed stop members 1-1 in the fourth grooves, the cuvette body 1 can be further fixed. Moreover, the cross-sectional area of the two fixed stop members 1-1 gradually decreases along the shell 2 from top to bottom, such that the sidewalls of the fixed stop members 1-1 allow more quickly sliding of the stop members into the designated positions of the fourth grooves.

Finally, it should be noted that the above embodiments are only used to illustrate examples of the technical solutions provided by the embodiments of the present application, and are not intended to limit them. Although the present application has been described in detail with reference to the foregoing embodiments, a person skilled in the art should understand that modifications may still be made to the technical solutions described in the foregoing embodiments, or some or all of the technical features may be equivalently replaced. These modifications or replacements do not cause the essence of the corresponding technical solutions to depart from the scope of the technical solutions of the embodiments of the present application.

## Claims

1. A medical flow-through cuvette, **characterized in that** the medical flow-through cuvette comprises:
a flow-through cuvette body comprising two fixed stop members and a side wall, wherein:
the two fixed stop members are arranged at intervals at an end of the flow-through cuvette body; and
the side wall of the flow-through cuvette body includes a planar structure between the two fixed stop members, and a cross-section of the flow-through cuvette body corresponding to the planar structure is different from another cross-section of the flow-through cuvette body adjacent to the planar structure, wherein a first side of the planar structure is configured to be adjacent to a detector that is outside of the flow-through cuvette body, the detector being configured to detect a liquid in the flow-through cuvette body.

2. The medical flow-through cuvette according to claim 1, wherein a cross-section of one of the fixed stop members is larger than a cross-section of an end of the flow-through cuvette body connected to the one of the fixed stop members.

3. The medical flow-through cuvette according to claim 1, wherein the flow-through cuvette body is provided with at least one first groove on the first side, the first groove being arranged along an extension direction of the medical flow-through cuvette, and the first groove being configured to be arranged on a periphery of the detector.

4. A medical blood liquid detection device, comprising:
the medical flow-through cuvette of any of claims 1-3; and
the detector comprising:
a shell including a first surface; and
a detection unit arranged in the shell, wherein the detection unit is configured to detect parameters of the liquid in the medical flow-through cuvette;
wherein the first surface of the shell is provided with a second groove and a fixing mechanism, the second groove being configured to receive the medical flow-through cuvette, one end of the fixing mechanism being movably connected to one side of the second groove, another end of the fixing mechanism being rotatable around the one end of the fixing mechanism, and the fixing mechanism covering a second surface of the medical flow-through cuvette.

5. The medical blood liquid detection device, further comprising a third groove in the second groove, wherein:
the cross-section of the flow-through cuvette body corresponding to the planar structure is larger than the another cross-section of the flow-through cuvette body; and
the planar structure cooperates with the third groove to fix the medical flow-through cuvette.

6. The medical blood liquid detection device, further comprising a boss in the second groove, wherein:
the cross-section of the flow-through cuvette body corresponding to the planar structure is smaller than the another cross-section of the flow-through cuvette body; and
the planar structure cooperates with the boss to fix the medical flow-through cuvette.

7. The medical blood liquid detection device according to claim 4, wherein:
the detection unit comprises a photoelectric transmitter and a photoelectric receiver;
the shell comprises a first chamber and a second chamber that are sequentially arranged along the first surface of the shell;
the first chamber is provided with the photoelectric transmitter; and
the second chamber is provided with the photoelectric receiver.

8. The medical blood liquid detection device according to claim 7, wherein:
the detection unit further comprises an infrared detector;
the shell further comprises a third chamber that is arranged adjacent to and spaced apart from the second chamber, wherein the infrared detector is provided in the third chamber; and
a first distance between the third chamber and the second chamber is different from a second distance between the second chamber and the first chamber.

9. The medical blood liquid detection device according to claim 7, wherein:
a first outer end of the first chamber includes a first transparent protective member that is configured to prevent foreign objects from falling into the first chamber;
a second outer end of the second chamber includes a second transparent protective member that is configured to prevent foreign objects from falling into the second chamber; and
a third outer end of the third chamber includes a third transparent protective member that is configured to prevent foreign objects from falling into the third chamber.

10. The medical blood liquid detection device according to claim 7, wherein:
a first length of the photoelectric transmitter and a second length of the photoelectric receiver along a blood flow direction of the medical flow-through cuvette are the same; and
a ratio of a center distance between the photoelectric transmitter and the photoelectric receiver to the first length is between 1.1 and 1.5.

11. The medical blood liquid detection device according to claim 6, further comprising an elastic member on a side of the fixing mechanism opposing the medical flow-through cuvette, wherein when the medical flow-through cuvette is fixed by the fixing mechanism, the elastic member contacts the medical flow-through cuvette and undergoes elastic deformation to secure the medical flow-through cuvette.

12. The medical blood liquid detection device according to claim 6, wherein cross-sections of the two stop members gradually decrease in an outward direction, the medical blood liquid detection device further comprising fourth grooves at ends of the shell at positions corresponding to the stop members, with a portion of the stop members being disposed in the corresponding fourth grooves to secure the medical flow-through cuvette.
